# EUROPEAN PATENT SPECIFICATION

(11) **EP 2 063 925 B1**
(45) Date of publication and mention of the grant of the patent: **24.02.2010**
(21) Application number: 07814067.0
(22) Date of filing: 14.08.2007
(51) Int. Cl.: A61L 31/14, A61L 31/10, A61L 31/16

(54) **STENT WITH ANTIMICROBIAL DRAINAGE LUMEN SURFACE**
STENT MIT ANTIMIKROBIELLER DRAINAGELUMEN-OBERFLÄCHE
STENT AVEC SURFACE DE LUMIÈRE DE DRAINAGE ANTIMICROBIENNE

(30) Priority: 28.08.2006 US 840595 P
(43) Date of publication of application: 03.06.2009
(73) Proprietor: Wilson-Cook Medical Inc., Winston-Salem, North Carolina 27105 (US)
(72) Inventor: RUCKER, Brian, K., King, NC 27021 (US)
(74) Representative: Garratt, Peter Douglas
(86) International application number: PCT/US2007/075880
(87) International publication number: WO 2008/027720

(56) References cited:
- WO-A-01/21229
- WO-A-99/38544
- US-A1- 2004 153 125
- US-A1- 2005 171 594
- US-A1- 2006 115 514

## Description

### TECHNICAL FIELD

The present disclosure relates to implantable medical devices. More particularly, the invention relates to fluid drainage devices, including drainage stents adapted for use in the biliary tract.

### BACKGROUND

Biliary stents can be implanted to maintain the patency of a biliary duct to treat various conditions, such as obstructive jaundice. Implanted biliary stents can provide for the palliation of malignant biliary obstruction, particularly when surgical cure is not possible. Biliary stenting can also be used to provide short-term treatment of conditions such as biliary fistulae or giant common duct stones. Long term implantation of biliary stents can be used to treat chronic conditions such as postoperative biliary stricture, primary sclerosing cholangitis and chronic pancreatitis.

A biliary stent can be advanced along a delivery catheter through an endoscope and deployed within a bile duct. The biliary stent may be configured as a tubular stent that is sufficiently strong to resist collapse of a body vessel, for example to maintain an open body vessel lumen through which digestive liquids can flow into the digestive tract. A biliary stent is also desirably longitudinally flexible enough to permit advancing the biliary stent along a path that may include sharp bends during delivery through and placement within a body vessel. The biliary stent is also desirably adapted to remain at a site of implantation within the bile duct without migrating after delivery.

Once implanted, an encrustation of amorphous biological material and bacteria ("sludge") may accumulate on the interior surface of the stent, gradually obstructing the lumen of the biliary stent and compromising drainage through the biliary stent. Biliary sludge is an amorphous substance often containing crystals of calcium bilirubinate and calcium palimitate, along with significant quantities of various proteins and bacteria. Sludge can deposit rapidly upon implantation in the presence of bacteria. For example, bacteria can adhere to plastic stent surfaces, using pili (hair-like projections from bacteria that allow bacterial cells to stick to surfaces) or through production of a mucopolysaccharide coating. The proliferation of bacteria within a glycocalyx matrix of the sludge within the lumen of an implanted biliary stent may form a biofilm. With time, an implanted biliary stent can thus become blocked, thereby restricting or blocking bile flow through the biliary stent. As a result, a patient can develop symptoms of recurrent biliary obstruction due to restricted or blocked bile flow through an implanted biliary stent, which can be complicated by cholangitis and sepsis. Often, such conditions are treated by antibiotics and/or endoscopic replacement of an obstructed biliary stent. However, the biofilm can provide a physical barrier protecting encased bacteria within the biliary stent lumen from antibiotics.

There exists a need in the art for an implantable medical device that prevents or reduces the biofilm and sludge deposition process inside the lumen of implantable drainage stents, such as biliary stents. Promising approaches have involved systemic administration of antibiotics, such as fluoroquinolone agents, that achieve high concentrations in bile and are effective against enteric Gram-negative bacteria. However, systemic treatment approaches may not allow penetration of the antibiotic agent through the glycocalyx matrix of biofilm that can insulate bacteria from contact with the antibiotic. U.S. Patent No. 6,887,270 describes implantable multilayer tubular medical devices incorporating antimicrobial agents in a matrix polymer around a barrier layer lining the drainage lumen. While the barrier layer may regulate the rate of release of the antimicrobial agent from the matrix polymer into the drainage lumen, the barrier layer is positioned between the antimicrobial agent and the drainage lumen and may prevent or reduce direct contact between the fluid in the drainage lumen and the antimicrobial agent.

Various antimicrobial materials may be adhered to an implantable medical device. For example, U.S. patent application publication US 2004/0153125 A1, filed December 4, 2003 by Roby describes coatings for an article of manufacture that include a metal salt of a fatty acid with antimicrobial properties, including a beryllium fatty acid salt. US 6,254,635, filed February 2, 1998 by Schroeder et al., describes deposition of an elemental metal such as beryllium on implantable biocompatible material (e.g., tissue heart valves) to reduce calcium deposition on the implanted material.

What is needed are implantable drainage stents having a lumen lined with a material configured to reduce the deposition of fluid and bacteria within the drainage lumen of the drainage stent, so as to prevent or reduce biodeposition within the drainage lumen.

### SUMMARY

The present disclosure provides a tubular drainage stent Including a support member circumferentially enclosing a drainage lumen at least partially defined by an antimicrobial coating. The antimicrobial coating is preferably configured to retain an antimicrobial material in contact with fluid within the drainage lumen. The antimicrobial material may comprise one or more materials selected from the group consisting of: beryllium, copper, cobalt, silicon and nickel. In one aspect, the coating comprises a coating layer comprising a porous material. For example, the antimicrobial material can be a metal or metal alloy with antimicrobial properties, optionally mixed with a porous biostable matrix polymer. The antimicrobial coating comprises beryllium, including beryllium alloys, or beryllium oxide. For example, a biostable antimicrobial coating may comprise an alloy containing between about 0.2% and 2.8% beryllium by weight. Examples of beryllium alloys useful in an antimicrobial coating include: Be-Cu, Be-Co-Cu, Be-Co-Si-Cu and Be-Ni-Cu. The antimicrobial material may optionally comprise other antimicrobial materials including: metallic silver, an alloy of silver containing about 2.5 wt % copper, silver citrate, silver acetate, silver benzoate, bismuth pyrithione, zinc pyrithione, zinc percarbonates, zinc perborates, bismuth salts, benzalkonium chloride (BZC) and sodium percarbonate.

An antimicrobial coating preferably has a porous surface defining a portion of the drainage lumen and typically has a tubular configuration. The antimicrobial coating may have two or more layers, including a porous diffusion layer positioned between the drainage lumen and the antimicrobial material. The porous diffusion layer defines open voids permitting fluid to pass through the porous diffusion layer to contact an antimicrobial material. The antimicrobial coating may have any suitable configuration, but is preferably configured to retain both an antimicrobial material and metal oxides formed by the antimicrobial material, including oxides of beryllium, copper and/or nickel. Preferably, the antimicrobial material is positioned to contact fluid within the drainage lumen of a drainage stent, preferably by defining at least a portion of the surface of the drainage lumen. For example, in one aspect, antimicrobial material may be incorporated into a portion of the support member lining a drainage lumen to form the antimicrobial coating. In a second aspect, the antimicrobial coating is a single-layer coating enclosed by a support member. For example, an antimicrobial material may be incorporated into a porous diffusion layer formed from a porous biostable polymer lining the drainage lumen of a tubular support member and defining at least a portion of the surface of a drainage lumen. In a third aspect, the antimicrobial coating comprises two or more coating layers. For example, the multilayer antimicrobial coating may be coated along the drainage lumen of a support member. The multilayer coating can comprise a first layer containing an antimicrobial material positioned between the support member and a second layer that defines at least a portion of a drainage lumen. The first layer and the second layer may be configured as concentric tubular coating layers adhered to or coextruded with the interior surface of a tubular support member. The second layer be configured as a porous layer to permit fluid from the drainage lumen to contact the antimicrobial material in the first layer, while retaining the metal oxides formed from the antimicrobial material. Suitable porous layer materials include a porous biostable polymer and/or a woven material such as expanded polytetrafluoroethylene (ePTFE).

A porous coating layer may have any suitable structure, but preferably has a void-volume percentage of between about 0.25 and 0.95, meaning the coating defines voids with a total volume of between about 25% and 95% of the total volume of the coating. The void can be open space, or can be filled with other materials. For example, a porous coating can comprise a biostable porous polymer defining voids totaling about 50% of the volume of the coating. These voids may optionally be filled with an antimicrobial material, an antimicrobial agent, or can be left open to permit fluid to flow through the voids within a body vessel.

Optionally, the antimicrobial coating may be adapted to release an antimicrobial agent into the drainage lumen. The releasable bioactive agent may be incorporated into one or more layers of an antimicrobial coating and/or the support member. Examples of antimicrobial agents include materials selected from the group consisting of: cephalosporins, clindamycin, chloramphenicol, carbapenems, minocyclines, rifampin, penicillins, monobactams, quinolones, tetracycline, macrolides, sulfa antibiotics, trimethoprim, fusidic acid, aminoglycosides, amphotericin B, azoles, flucytosine, cilofungin, nikkomycin Z, and rifamycin.

The support member may be configured in any manner suitable for a desired use. The support member preferably houses a drainage lumen adapted to permit fluid flow therethrough, and encloses an antimicrobial coating lining at least a portion of the drainage lumen. The support member may have any suitable configuration that provides a desired level of flexibility or rigidity for an intended application. For a biliary stent, the support member should be flexible enough to permit endoscopic delivery, yet rigid enough to maintain an open passageway within a biliary duct. For stenting applications, such as biliary stents, the support member is typically an annular tubular structure with an interior surface. The interior surface can be coated with an annular antimicrobial coating, which may define a tubular drainage lumen extending longitudinally through the support member. Other examples of suitable support member configurations may include an undulating or roughened interior surface, or a corrugated or grooved exterior surface along the drainage lumen. The support member is preferably formed from any suitable material, such as a polyethylene and/or polyurethane, that is thermoformable, biocompatible and provides desired levels of rigidity and adherence by the antimicrobial material. The antimicrobial coating can be attached to, or incorporated within, the support member in any suitable manner, including co-extrusion with the support member, dipping of the support member in a solution of an antimicrobial material, or coating the antimicrobial material onto the lumen surface of the drainage stent.

### BRIEF DESCRIPTION OF THE DRAWINGS

**Figure 1** is a side view of a first biliary drainage stent embodiment.

**Figure 2** is a longitudinal cross sectional view of a portion of the biliary stent along the line A-A' shown in **Figure 1****.**

**Figure 3** is a transverse cross sectional view of a portion of the biliary stent along the line B-B' shown in **Figure 1****.**

**Figure 4** is a transverse cross sectional view of a portion of an alternative biliary stent coating configuration, similar to the biliary stent shown in **Figure 1****.**

**Figure 5** is a side view of a second biliary drainage stent embodiment having a bent support member configuration.

**Figure 6** is a side view of a third biliary drainage stent embodiment having a pigtail support member configuration.

### DETAILED DESCRIPTION

The invention provides medical devices for implantation in a body vessel.

### Definitions

As used herein the terms "comprise(s)," "include(s)," "having," "has," "contain(s)," and variants thereof, are intended to be open-ended transitional phrases, terms, or words that do not preclude the possibility of additional acts or structures.

As used herein, the term "body vessel" means any body passage lumen that conducts fluid, including but not limited to biliary ducts, ureteral passages, esophagus, and blood vessels such as those of the human vasculature system.

As used herein, the term "implantable" refers to an ability of a medical device to be positioned at a location within a body, such as within a body vessel. Furthermore, the terms "implantation" and "implanted" refer to the positioning of a medical device at a location within a body, such as within a body vessel.

As used herein, "endolumenally," "intralumenal" or "transluminal" all refer synonymously to implantation placement by procedures wherein the medical device is advanced within and through the lumen of a body vessel from a remote location to a target site within the body vessel. Endolumenal delivery includes implantation in a biliary duct from an endoscope or catheter.

A "biocompatible" material is a material that is compatible with living tissue or a living system by being consistent with an intended course of treatment, including not being undesirably toxic or injurious and not causing an undesirable immunological rejection.

The terms "biostable" and "non-bioabsorbable" material refer synonymously to a material, such as a polymer or copolymer, which remains in the body without substantial bioabsorption or dissipation.

The term "bioabsorbable" is used herein to refer to materials selected to dissipate upon implantation within a body, independent of which mechanisms by which dissipation can occur, such as dissolution, degradation, absorption and excretion. The actual choice of which type of materials to use may readily be made by one ordinarily skilled in the art. Such materials are often referred to by different terms in the art, including "bioresorbable," "bioabsorbable," or "biodegradable," depending upon the mechanism by which the material dissipates. As used herein, "bioabsorbable polymer" refers to a polymer or copolymer which dissipates upon implantation within the body. A large number of different types of materials are known in the art which may be inserted within the body and later dissipate.

The term "void-to-volume" refers to the volume of the pores of a porous material divided by the total volume of the material including the volume of the pores. Void-to-volume can be measured using the protocol described in AAMI (Association for the Advancement of Medical Instrumentation) VP20-1994, Cardiovascular Implants--Vascular Prosthesis section 8.2.1.2, Method for Gravimetric Determination of Porosity. The porosity of the porous material may also be observed by a scanning electron microscope (SEM) image.

Various medical devices for implantation in a body vessel are disclosed herein. Preferred embodiments relate to a medical device comprising an antimicrobial coating positioned within a support member such that at least a portion of the antimicrobial coating forms the lining of a drainage lumen. The medical devices are described with respect to an exemplary biliary stent embodiment comprising a substantially tubular support member having a drainage lumen at least partially lined with an antimicrobial coating. However, other medical devices, such as ureteral stents, esophageal stents or catheters, can also be used as implantable medical devices according to other embodiments.

### Medical Device Configurations

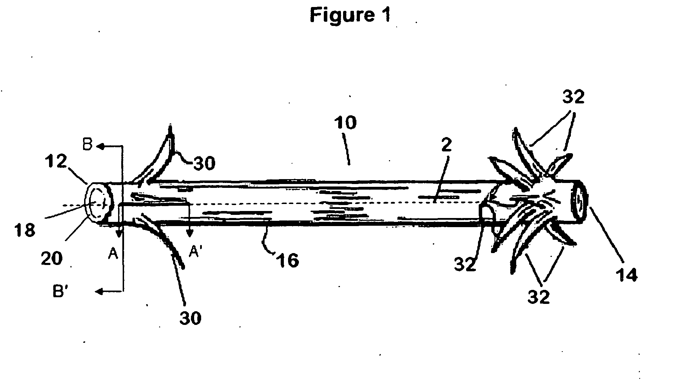

**Figure 1** is a side view of an exemplary implantable medical device configured as a drainage stent 10. The drainage stent 10 is a biliary drainage stent having a drainage tube 16 housing an internal drainage lumen 18 extending from an inlet 14 to an outlet 12. The drainage tube 16 has an interior surface 20 defining the inlet 14 and outlet 12. Inlet 14 permits fluid to enter the drainage tube 16, while outlet 12 defines an opening permitting the fluid to exit the drainage tube 16. Unless otherwise indicated, the terms "inlet" and "outlet" are used in an exemplary manner to refer to the antegrade direction of fluid flow through a medical device into the medical device through the inlet and exiting the medical device from the outlet, but do not preclude reverse (retrograde) fluid flow in the opposite direction, or bidirectional flow in both antegrade and retrograde directions. Typically, medical devices are implanted to permit fluid to flow through the medical device in substantially the antegrade direction. The drainage stent 10 comprises one inlet 14 and one outlet 12. Other embodiments provide a medical device with multiple inlets and/or outlets, including inlets or outlets placed along the side of a drainage tube.

The drainage stent 10 is preferably configured for placement within a biliary or pancreatic duct with the drainage stent 10 extending along the length of the duct and the outlet 12 extending into the duodenum. While the preferred embodiment describes a drainage stent 10 intended for use in the common bile duct or pancreatic duct of a patient having a ductal occlusion or obstruction, the drainage stent 10 may also be configured for use in other areas within the body. For example, the stent could be configured for use within a ureteral, urethral, esophageal or blood vessel.

Preferably, the medical device comprises a means for anchoring the device within a body passage, such as a plurality of anchoring flaps extending radially outward from the exterior surface of the inlet 14 or outlet 12 portions of the drainage stent 10. The number, size and orientation of anchoring flaps can be modified to accommodate the migration-preventing requirements of the particular medical device to be implanted, the site of implantation and the desired function of the device. For example, the drainage stent 10 comprises an outlet array 30 and an inlet array 32 of radially extending flaps attached to the exterior surface of the drainage tube 16 proximate to the outlet 12 and the inlet 14, respectively, to anchor drainage stent 10 within a biliary duct. The anchoring flaps can be formed by slicing small longitudinal sections in the distal or proximate ends of the drainage tube 16 and orienting the sliced sections radially. Preferably, the slice incisions are made in the exterior surface of the drainage tube 16 in a shallow manner so as to not create holes in the drainage tube 16.

The drainage tube 16 can be substantially straight and symmetrically disposed about a longitudinal axis 2, as shown in Figure 1. The drainage tube 16 may have any suitable diameter for an intended use. In a biliary drainage stent 10, the drainage tube 16 may have an external diameter of between about 7-12 French 2.31mm - 3.96mm (0.091 - 0.156 inches) and a length of between about 25 - 180 mm between the inlet 14 and the outlet 12.

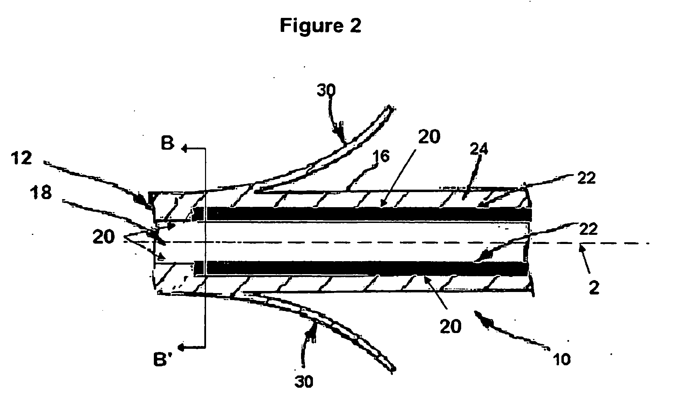

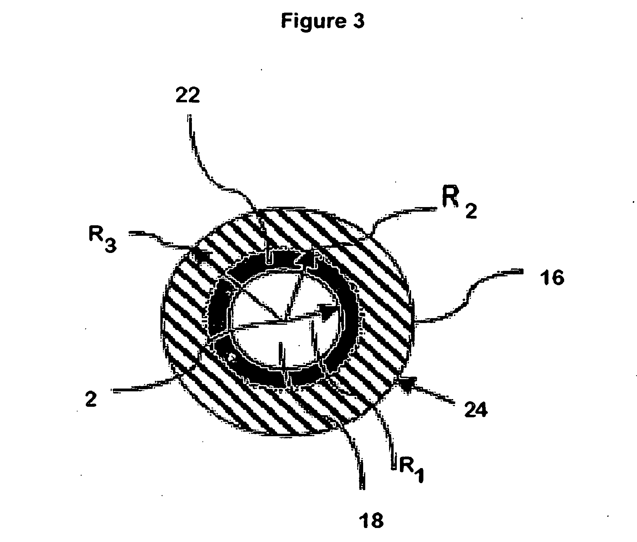

**Figure 2** shows a longitudinal cross section of the drainage stent 10 along the line A-A' in **Figure 1****;** **Figure 3** shows a transverse cross section of the drainage stent 10 along the line B-B' in **Figure 1** and **Figure 2****.** Referring to **Figure 2****,** the drainage tube 16 houses a drainage lumen 18 centered along the longitudinal axis 2 of the drainage stent 10. The antimicrobial coating 22 preferably forms at least a portion of the perimeter of the drainage lumen 18, and can be adhered directly to the interior surface of the support member 24. The drainage tube 16 preferably includes a support member 24 and an antimicrobial coating 22. The antimicrobial coating 22 is enclosed by the internal surface of the support member 24. The support member 24 can be configured as a continuous uninterrupted tube adapted to provide a drainage lumen through an obstructed portion of a body vessel, such as a biliary duct. Alternatively, the support member 24 can be have a bifurcated tubular configuration and/or comprise one or more additional inlet or outlet orifices between the drainage lumen and the exterior surface along the length of a tubular body. Preferably, the support member 24 forms the exterior surface of the drainage tube 16, although the drainage tube 16 may include additional layers of material placed on the exterior surface of the support member 24. The support member 24 may have one or more layers of material. The support member 24 has an interior surface 20. The interior surface 20 preferably defines distal and proximal portions of the drainage lumen 18 at the outlet 12 and the inlet 14. The drainage lumen 18 extends from the inlet 14 and the outlet 12, and may be defined by the antimicrobial coating 22. The antimicrobial coating 22 may define a portion of the drainage lumen 18 (as shown in Figure 2), or may define the entire drainage lumen 18 by extending to the inlet 14 and the outlet 12 of the support member 24. Preferably, the antimicrobial coating 22 is configured to retain the antimicrobial material. Optionally, the interior surface 20 of the support member 24 may be adapted to promote adhesion of the antimicrobial coating 22, for example by roughening, plasma treatment or by applying an adhesion promoting material such as silane or parylene. Alternatively, the support member 24 and the antimicrobial coating 22 may be formed together by a method such as coextrusion or solvent casting.

The radial thickness of the drainage tube 16 and the support member 24 can be selected to provide a drainage stent 10 with a desired amount of flexibility or rigidity for an intended application. Referring to **Figure 3****,** the outer radius R₃ of the drainage tube 16 is measured as the radial distance from the longitudinal axis 2 to the exterior surface of the drainage tube 16. R₁ is the radius of the drainage lumen 18 and R₂ is the radius from the longitudinal axis 2 to the interior surface of the support member 24. The outer radius R₃ is greater than the radius R₁ of the interior drainage lumen 18. The thickness of the support member 24 is R₃-R₂. The thickness of drainage tube 16 depends on the material selected, and can be any thickness providing a desired amount of radial support, while retaining a desired level of flexibility. For example, a polyethylene biliary stent support member 24 typically has a drainage tube 16 with a thickness of about 0.2 mm to about 1.0 mm, preferably about 0.4 mm for a 10F stent. Typical values for the radius R₁ of a drainage lumen 18 for a biliary stent can vary from about 0.25 mm to about 1.5 mm, including about 0.5 mm to about 1.5 mm for a 10 F stent to about 0.25 mm to about 0.75 mm for a 5F stent. The radial thickness and composition of the antimicrobial coating 22 (i.e., R₂-R₁ in **Figure 3**) can be selected to provide a desired rate of fluid flow through a drainage lumen 18, and to prevent or reduce the biodeposition of biomaterial. For biliary stents, biomaterial can include the components of a glycocalyx matrix or bacteria. For vascular stents, biomaterial can include blood components active in thrombus formation. The radial thickness of the antimicrobial coating 22 (R₂-R₁) is typically about 0.2 mm to about 0.5 mm.

Alternatively, the antimicrobial coating 22 can be combined with the support member 24 (not shown), for example by coextrusion of an exterior tube of polyethylene with an interior annular coating layer comprising an antimicrobial material and a porous biostable material configured to retain the antimicrobial material in the coating layer. The support member 24 may also be formed as a single thermoformable layer of material comprising the antimicrobial material. The antimicrobial material may be homogeneously mixed within the support member 24, or may be present in a radial concentration gradient within the support member 24. Where the antimicrobial coating 22 is combined with the support member 24, R₂ is absent and the thickness of the combined antimicrobial coating 22 and the support member 24 are both equal to R₃-R₁, and can be about 0.2 mm to about 1.0 mm. Therefore, the total radial thickness of the drainage tube 16 and the antimicrobial coating 22 are desirably between about 0.4 mm to about 0.8 mm. The relative radial thicknesses of the drainage tube 16 and the antimicrobial coating 22 can be selected to provide a minimal desired amount of radial strength to maintain patency of the drainage lumen 18 upon implantation.

Referring again to **Figure 3****,** where the drainage tube 16 includes a separate antimicrobial coating 22 enclosed by a support member 24, the radial thickness of the support member 24 (R₃ - R₂) and the antimicrobial coating 22 (R₂ - R₁) can be varied. The ratio of the radial thickness of the antimicrobial coating 22 (R₂-R₁) to the radial thickness of the drainage tube 16 (R₃-R₂) is preferably less than about 20:1 - more preferably less than about 10:1, 5:1, 3:1 or 2:1 and most preferably about 1:1. The ratio of the radius of the drainage lumen 18 to the radial thickness of the antimicrobial coating 22 is preferably less than about 5:1 - more preferably about 2:1.

The surface of the antimicrobial coating 22 defining the drainage lumen 18 is preferably configured to maximize the surface area of the antimicrobial coating 22 lining the drainage lumen 18. The antimicrobial coating 22 preferably has a roughened or pitted surface. Roughening of the surface can desirably provide increased contact between fluid in the drainage lumen 18 and the antimicrobial material of the antimicrobial coating 22. Optionally, the surface of the antimicrobial coating 22 defining the drainage lumen 18 may have a porous or mesh-like morphology and can be permeable to fluid from the drainage lumen 18. Alternatively, the antimicrobial coating 22 surface can be extruded with a grooved or pitted surface to provide a desired surface roughness.

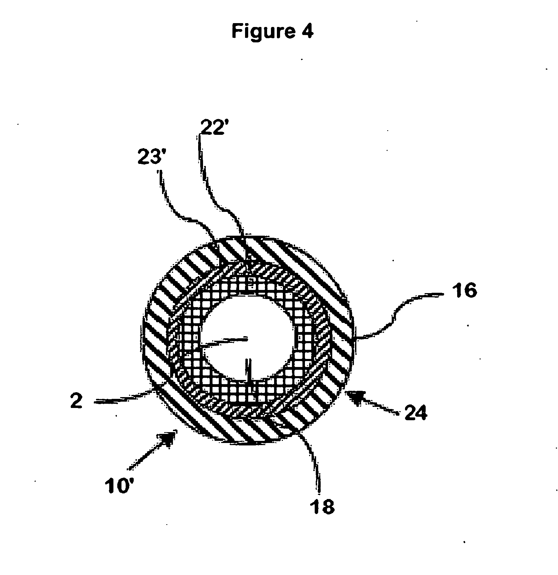

The antimicrobial coating 22 can be configured with one or more coating layers. The drainage stent 10 shown in the embodiment of **Figures 1-3** comprises a single-layer antimicrobial coating 22. Alternatively, the antimicrobial coating 22 can also be a multilayer coating. **Figure 4** shows a transverse cross section of a drainage stent similar to the drainage stent 10' shown in **Figure 1****,** except that the drainage stent 10' includes a two-layer antimicrobial coating. The two-layer antimicrobial coating is circumferentially enclosed by a support member 24. The antimicrobial coating comprises a first layer 22' defining the drainage lumen 18 enclosing the longitudinal axis 2 of the drainage stent, and a second layer 23' positioned between the first layer 22' and the support member 24. The first layer 22' may be configured as a porous biostable material, as described below, and may optionally contain an antimicrobial material and/or an antimicrobial bioactive agent. Optionally, the first layer 22' is impregnated with a releasable antimicrobial bioactive agent. The porosity of the first layer 22' is preferably selected to permit fluid in the drainage lumen 18 to pass through the first layer 22' and contact the second layer 23', and to retain metal oxides formed in the second layer 23', which may include beryllium, nickel and/or copper oxides. The second layer 23' preferably comprises an antimicrobial material including beryllium, nickel and/or copper.

### Antimicrobial Coatings

The antimicrobial coating (e.g., 22 or (22', 23')) preferably includes one or more layers comprising an antimicrobial material. The antimicrobial coating will be discussed further with reference to the exemplary one-layer antimicrobial coating 22, although the description of the antimicrobial coating 22 may also apply to one or more layers such as 22' and/or 23'. Preferably, the antimicrobial coating 22 is enclosed within a drainage stent and includes a porous biostable material in contact with the antimicrobial material. The biostable material may be mixed with or layered over the antimicrobial material. The biostable material is preferably configured to retain the antimicrobial material after implantation in contact with fluid within the drainage lumen 18. For example, biostable materials forming a porous network with a low void-volume percentage may be desirable for retaining an antimicrobial material within the antimicrobial coating 22. The penetration of fluid within the drainage lumen 18 into the antimicrobial coating 22 may be increased by increasing the pore size of the material in the antimicrobial coating 22. In one embodiment, a porous antimicrobial coating 22 may have a void-volume percentage of 25% to 95% (0.25 to 0.95). In another embodiment, the porous second layer has a void-volume percentage of 40% to 90%. The pores of the antimicrobial coating 22 desirably have an average pore diameter of 1 micron to 400 microns. Typically, the antimicrobial material is dispersed in a matrix of a biostable material, such as a porous biocompatible biostable polymer. Optionally, one or more layers of the antimicrobial coating 22 can include a soluble material that is readily dissolvable upon contact with fluid in the drainage lumen, such as a water-soluble polymer. The soluble material is preferably mixed with a porous biostable material and an antimicrobial material to form one or more layers of the antimicrobial coating 22. Upon implantation, the soluble material can dissolve into fluid within the drainage lumen 18 and be carried away through the outlet 12, leaving a porous antimicrobial coating layer comprising the antimicrobial material and a porous biostable material to retain the antimicrobial material within the stent.

The biostable material should be strong enough to withstand mechanical stress or strain anticipated during delivery and upon implantation within the body vessel. The biostable material can include any suitable material selected to adhere to the interior surface of the support member 24, or can be formed from the same material as the support member 24. Preferably, the biostable material is a polymer having a molecular weight that is high enough to provide sufficient durability so that the polymer will not be rubbed off during sterilization, handling, or deployment of the medical device and will not crack when the device is expanded. Exemplary polymer systems that may also be used as a biostable material in one or more coating layers include biocompatible polymers. Suitable biostable materials include: polyethylene, polyurethane, an ethylene vinyl acetate copolymer, copolymers of ethylene with acrylic acid or methacrylic acid, metallocene-catalyzed polyethylenes (mPE), and polyethylene copolymers, ionomers, elastomeric materials such as elastomeric polyurethanes and polyurethane copolymer, silicones and mixtures thereof. The biostable material can be a porous EVA copolymer having a vinyl acetate content of between 3% and 28% by weight, wherein increasing the vinyl acetate content of an EVA copolymer may increase the permeability of the copolymer. Other representative examples of suitable biostable materials include ethylene-covinyl acetate copolymers, acrylic-based and methacrylic-based polymers (e.g., poly(acrylic acid), poly(methylacrylic acid), poly(methylmethacrylate), poly(hydroxyethyl methacrylate), poly(alkylcynoacrylate), poly(alkyl acrylates), poly(alkyl methacrylates)), polyolefins (e.g., poly(ethylene) or poly(propylene)), polyamides (e.g., nylon 6,6), poly(urethanes) (e.g., poly(ester urethanes), poly(ether urethanes), poly(carbonate urethanes), poly(ester-urea urethanes)), polyesters (e.g., PET, polybutyleneterephthalate, and polyhexyleneterephthalate), olyethers (e.g., poly(ethylene oxide), poly(propylene oxide), poly(ethylene oxide)-poly(propylene oxide) copolymers, diblock and triblock copolymers), and poly(tetramethylene glycol)), silicone containing polymers and vinyl-based polymers (e.g., polyvinylpyrrolidone, poly(vinyl alcohol), poly(vinyl acetate phthalate), and poly(styrene-co-isobutylene-co-styrene)), and fluorine containing polymers (fluoropolymers) (e.g., fluorinated ethylene propylene (FEP) and polytetrafluoroethylene (PTFE) and expanded polytetrafluoroethylene (ePTFE)). Expanded polytetrafluoroethylene, polyurethane and polyethylene are particularly preferred biostable materials, suitable for combination with an antimicrobial material to form an antimicrobial coating 22.

The antimicrobial coating 22 may have any suitable configuration or morphology. Preferably, the antimicrobial coating 22 comprises a biostable polymer in the form of a mesh impregnated with an antimicrobial material. Alternatively, the mesh may be positioned over a layer of antimicrobial material. A mesh typically includes a plurality of fibers or filaments (i.e., a fibrous material), where the fibers or filaments are arranged in such a manner (e.g., interwoven, knotted, braided, overlapping, looped, knitted, interlaced, intertwined, webbed, felted, and the like) so as to form a porous fibrous network. Typically, a mesh is a pliable material. The mesh may be capable of providing support to the structure (e.g., the vessel or cavity wall) and may be adapted to release an amount of the antimicrobial agent. Mesh materials may take a variety of forms. The mesh preferably comprises interstices large enough to permit fluid in the drainage lumen 18 to contact the antimicrobial material, but small enough to prevent the antimicrobial material from entering the drainage lumen 18. Typically, the mesh possesses sufficient porosity to permit the flow of fluids through the interstices and to facilitate contact between the antimicrobial material and fluid in the drainage lumen 18. The flow of fluid through the interstices of the mesh depends on a variety of factors. The porosity of the mesh may be further tailored by, for example, filling the interstices of the mesh with another material (e.g., particles or polymer) that is soluble in the fluid passing through the drainage lumen, or by processing the mesh (e.g., by heating) in order to reduce the pore size and to create non-fibrous areas. Fluid flow through the mesh of the invention will vary depending on the properties of the fluid, such as viscosity, hydrophilicity/hydrophobicity, ionic concentration, temperature, elasticity, particulate content, and the like. Preferably, the interstices prevent the release of impregnated or coated antimicrobial materials from the antimicrobial coating 22, while permitting fluid to contact the antimicrobial materials within the mesh. The antimicrobial coating 22 may be formed from porous ePTFE tubing composed of a microstructure of nodes interconnected by fibrils. See, e.g., U.S. Patent Nos. 5,152,782 and 4,955,899. The antimicrobial coating 22 may also be a plurality of polymeric fibers knitted together composed of one or more fibers.

Preferably, the antimicrobial coating 22 comprises a biostable material mixed with an antimicrobial material selected to reduce or eliminate the adhesion of bacteria on the surface the antimicrobial coating 22 defining the drainage lumen 18. The antimicrobial material may comprise beryllium and one or more materials selected from the group consisting of: copper, cobalt, silicon and nickel, particularly materials disclosed in U.S. Patent No. 5,423,631 to Inoue. Accordingly, certain alloys comprising beryllium, copper and/or nickel having an antifouling effect in seawater may also reduce the incidence of biofilm deposition within the drainage lumen 18 of a drainage stent 10. The antimicrobial coating comprises beryllium, including beryllium alloys or beryllium oxide.

References to alloy compositions provided as "X-Y," where X and Y are alloy components, refer to alloy compositions comprising components X and Y in any relative amount, and having X and Y in any oxidation state. The alloy composition may also include oxide products of X and Y in any stoichiometry. For example, a Be-Cu alloy contains any suitable amount of Be and Cu in an ionized or non-ionized form, and in any stoichiometry, unless otherwise specified. Accordingly, a biostable antimicrobial coating 22 may comprise an alloy containing between about 0.2% and 2.8% beryllium by weight. Examples of beryllium alloys within an antimicrobial coating include: Be-Cu, Be-Co-Cu, Be-Co-Si-Cu and Be-Ni-Cu. Particularly preferred antimicrobial material compositions comprise one or more copper alloys selected from the group consisting of: (1) 0.2 to 1.0% by weight of beryllium, 2.4 to 2.7% by weight of cobalt and the balance being copper; (2) 0.2 to 1.0% by weight of beryllium, 1.4 to 2.2% by weight of nickel and the balance being copper; (3) 1.0 to 2.0% by weight of beryllium, 0.2 to 0.6% by weight of cobalt and the balance being copper; and (4) 1.6 to 2.8% by weight of beryllium, 0.4 to 1.0% by weight of cobalt, 0.2 to 0.35% by weight of silicon and the balance being copper.

Preferably, antimicrobial agents comprise one or more materials selected from the group consisting of: beryllium (Be), cobalt (Co), nickel (Ni) and silicon (Si), incorporated individually or in combination in a copper alloy antimicrobial material within the following ranges:
Beryllium--0.2 to 2.8% by weight
Cobalt--0.2 to 2.7% by weight
Nickel--1.4 to 2.2% by weight
Silicon--0.2 to 0.35% by weight.
Cobalt may be provided as a CoBe compound dispersed throughout an alloy or a biostable polymer matrix. Nickel may be provided to form a fine NiBe compound dispersed throughout the alloy matrix, thereby improving the mechanical properties and productivity of the copper alloy. The order of ionization tendency among beryllium, copper and nickel is Be>Ni>Cu. Therefore, beryllium is ionized more readily than nickel or copper, respectively, leading to formation of a beryllium oxide (BeO) material within the antimicrobial material. This BeO material is typically porous, and may allow copper ions to be formed as Cu₂O and BeO within the antimicrobial material. Preferably, the antimicrobial material is contained within a matrix of biostable polymer impregnated with the antimicrobial material and configured to retain Cu₂O and BeO materials within a microporous biostable structure. Antimicrobial materials comprising nickel may also form nickel oxide (NiO₂). According to the order of ionization tendency (Be>Ni>Cu), nickel (Ni) is preferentially ionized compared to copper. Accordingly, the formation of nickel oxide may reduce the quantity of the copper oxide formed within the antimicrobial material.

Other examples of suitable antimicrobial materials include nanosize particles of metallic silver or an alloy of silver containing about 2-5 wt% copper (hereinafter referred to as "silver-copper"), salts such as silver citrate, silver acetate, silver benzoate, bismuth pyrithione, zinc pyrithione, zinc percarbonates, zinc perborates, bismuth salts, various food preservatives such as methyl, ethyl, propyl, butyl, and octyl benzoic acid esters (generally referred to as parabens), citric acid, benzalkonium chloride (BZC), rifamycin and sodium percarbonate. Another suitable antimicrobial material is described in published U.S. patent application US2005/0008763A1 (filed September 23, 2003 by Schachter).

The antimicrobial coating 22 may optionally include one or more antimicrobial agents. The term "antimicrobial agent" refers to a bioactive agent effective in the inhibition of, prevention of or protection against microorganisms such as bacteria, microbes, fungi, viruses, spores, yeasts, molds and others generally associated with infections such as those contracted from the use of the medical articles described herein.
The antimicrobial agents include antibiotic agents and antifungal agents. Antibiotic agents include cephalosporins, clindamycin, chloramphenicol, carbapenems, penicillins, monobactams, quinolones, tetracycline, macrolides, sulfa antibiotics, trimethoprim, fusidic acid and aminoglycosides. Antifungal agents include amphotericin B, azoles, flucytosine, cilofungin and nikkomycin Z. Other non-limiting examples of suitable antibiotic agents include: ciprofloxacin, doxycycline, amoxicillin, metronidazole, norfloxacin (optionally in combination with ursodeoxycholic acid), ciftazidime, and cefoxitin. Bactericidal nitrofuran compounds, such as those described by U.S. Patent No. 5,599,321 (Conway et al.), can also be used as an antimicrobial agent. Preferred nitrofuran antimicrobial agents include nitrofurantoin, nitrofurazone, nidroxyzone, nifuradene, furazolidone, furaltidone, nifuroxime, nihydrazone, nitrovin, nifurpirinol, nifurprazine, nifuraldezone, nifuratel, nifuroxazide, urfadyn, nifurtimox, triafur, nifurtoinol, nifurzide, nifurfoline, nifuroquine, and derivatives of the same, and other like nitrofurans which are both soluble in water and possess antibacterial activity. References to each of the above-cited nitrofuran compounds may be found in the Merck Index, published by Merck & Co., Inc., Rahway, N.J..
Other suitable antimicrobial agents include rifampin, minocycline, novobiocin and combinations thereof discussed in U.S. Patent No. 5,217,493 (Raad et al.). Rifampin inhibits bacterial DNA-dependent RNA polymerase activity and is bactericidal in nature, and is available in the United States from Merrill Dow Pharmaceuticals, Cincinnati, Ohio. Minocycline is a bacteriostatic semisynthetic antibiotic derived from tetracycline, and is commercially available from Lederle Laboratories Division, American Cyanamid Company, Pearl River, N.Y. Novobiocin is an antibiotic with bacteriostatic action that appears to interfere with bacterial cell wall synthesis and inhibits bacterial protein and nucleic acid synthesis and to affect stability of the cell membrane by complexing with magnesium. Novobiocin is available from The Upjohn Company, Kalamazoo, Michigan.

In one aspect, the antimicrobial coating does not include or is substantially free of biologically reactive components, such as a tissue, biological material, or any collagen-based explanted tissue or extracellular matrix material. In another aspect, the antimicrobial coating is substantially free of a biodegradable polymer or other biodegradable material. The antimicrobial coating preferably includes a biostable polymer and beryllium or a beryllium metal alloy.

Unless otherwise indicated, a beryllium-containing antimicrobial coating refers a composition including beryllium in the elemental and/or ionized state, including beryllium salts and complexes. Optionally, the antimicrobial coating may include an oxide or hydroxide salt of beryllium. For example, beryllium hydroxide (Be(OH)₂) and/or beryllium ion complexes (e.g., [Be(OH)₄]²⁺_{(aq)}, [Be(OH)₄]²⁻_{(aq)} and [Be(H₂O)₄]²⁺_{(aq)}) may be present in the antimicrobial coating before or after implantation a medical device with the antimicrobial material. Other beryllium ion salts may also be included in the antimicrobial coating, such as Be(H₂O)₂(OH)₂₍ₛ₎.

### Support Members

The support member 24 can be made from any biocompatible material that is resiliently compliant enough to readily conform to the curvature of the duct in which it is to be placed, while having sufficient "hoop" strength to substantially retain its form within the duct. Preferably, the support member 24 is formed from a biocompatible polyethylene. Other suitable materials for the support member 24 include polyurethane (such as a material commercially available from Dow Corning under the tradename PELLETHANE), a silicone rubber (such as a material commercially available from Dow Corning under the tradename SILASTIC), a potyetheretherketone (such as a material commercially available from Victrex under the tradename PEEK). The support member 24 can be formed from any suitable biocompatible and non-bioabsorbable material. Preferably, the support member 24 is formed from a thermoformable material that can be coextruded with an antimicrobial material, with the antimicrobial material 22 and the support member 24 in the same or separate layers. One suitable support member 24 with a substantially straight configuration shown in Figure 1 is the COTTON-LEUNG® (Amsterdam) Biliary Stent (Cook Endoscopy, Winston-Salem, North Carolina, USA).

Preferably, the support member 24 is formed from a polyolefin such as a metallocene catalyzed polyethylene, polypropylene, polybutylene or copolymers thereof. Other non-limiting examples of biostable polymers useful for manufacturing the support member 24 include: vinyl aromatic polymers (e.g., polystyrene), vinyl aromatic copolymers (e.g., styrene-isobutylene copolymers and butadiene-styrene copolymers), ethylenic copolymers (e.g., ethylene vinyl acetate (EVA)), ethylene- methacrylic acid and ethylene- acrylic acid copolymers, ionomers, polyacetals, chloropolymers (e.g., polyvinylchloride (PVC)), polyesters (e.g., polyethyleneterephthalate (PET)), polyester-ethers, polyamides (e.g., nylon 6 and nylon 6,6), polyamide ethers, polyethers, elastomers (e.g., elastomeric polyurethanes and polyurethane copolymers), silicones, and polycarbonates, as well as mixtures and block or random copolymers of any of the foregoing.

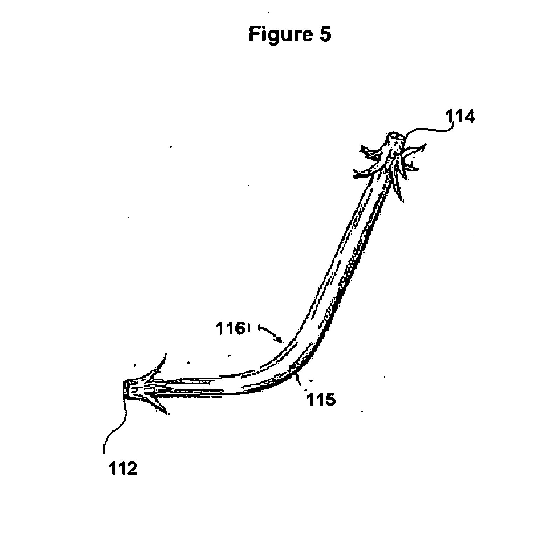

The support member 24 may also have one or more bends, as shown in **Figure 5****.** In one aspect, drainage tube 116 includes a bend 115 positioned about mid-way between the outlet 112 and the inlet 114, so as to accommodate the anatomical structure of a biliary duct. The bend preferably conforms to the duodenal anatomy, and can be about 120 degrees. Alternatively, the bend can be positioned about 1/3 of the distance from the inlet 114 and the outlet 112. Examples of suitable support members 24 having a bent configuration shown in **Figure 5** include: COTTON-HUIBREGTSE® Biliary Stents, COTTON-LEUNG® (Amsterdam) Stents, GEENEN® Pancreatic Stents, ST-2 SOEHENDRA TANNENBAUM Biliary Stents and JOHLIN® Pancreatic Wedge Stents, all commercially available from Cook Endoscopy (Winston-Salem, North Carolina, USA).

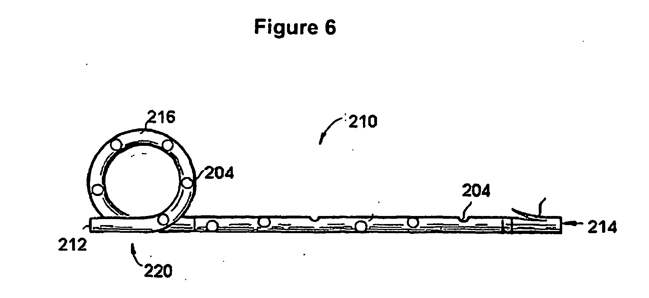

Referring to **Figure 6****,** a medical device 210 can include support member with a "pigtail" configuration, where the drainage tube 216 comprises a plurality of drainage holes 204. One or both ends of the drainage tube 216 can be curled to form a planar loop 220. In addition to the inlet 214 and outlet 212, fluid can enter or exit the lumen through the plurality of drainage holes 204 along the drainage tube 216. For example, the drainage tube 216 may have a drainage stent configuration described in U.S. Patent 5,052,998, filed April 4, 1990 by Zimmon. The antimicrobial coating can be applied discontinuously along the lumen of the drainage tube 216, without covering the drainage holes 204. The drainage tube 216 may have any suitable diameter. Preferably, the external diameter of the drainage tube 216 is shaped and configured for implantation within a biliary duct. Examples of suitable stents 210 having a coiled ("pigtail") inlet and outlet configuration shown in **Figure 6** include: Double Pigtail Stent, the ZIMMON® Biliary Stent and the ZIMMON® Pancreatic Stents, all commercially available from Cook Endoscopy (Winston-Salem, North Carolina, USA).

The support member, or other portion of the stent, may be provided with marker bands at one or both of the distal and proximal ends. The marker bands (not shown) may be formed from a suitably radiopaque material. The marker bands can provide a means for orienting the stent within a body vessel. The marker band, such as a radiopaque portion of the support member, can be identified by remote imaging methods including X-ray, ultrasound, Magnetic Resonance imaging, fluoroscope and the like, or by detecting a signal from or corresponding to the marker band. In other embodiments, a device for delivering the drainage stent can comprise radiopaque indicia relating to the orientation of the support member within the body vessel. A stent or delivery device may comprise one or more radiopaque materials to facilitate tracking and positioning of the medical device, which may be added in any fabrication method or absorbed into or sprayed onto the surface of part or all of the stent. For example, radiopaque markers can be used to identify a long axis or a short axis of a medical device within a body vessel. For instance, radiopaque material may be attached to a support member or woven into portions of the drainage stent or other medical device. The degree of radiopacity contrast can be altered by changing the composition of the radiopaque material. For example, radiopaque material may be covalently bound to the support member. Common radiopaque materials include barium sulfate, bismuth subcarbonate, and zirconium dioxide. Other radiopaque materials include: cadmium, tungsten, gold, tantalum, bismuth, platinum, iridium, iodine and rhodium.

### Methods of Manufacture

The medical devices can be formed in any suitable manner that provides a structure having a support member 24 enclosing an antimicrobial coating 22 defining-at least a portion of the surface of a drainage lumen 18. The support member 24 is preferably a thermoformable, biostable material providing a desired level of mechanical strength to the medical device.

Preferably, the support member 24 and the antimicrobial coating 22 are formed together by co-extrusion. Preferred coextrudable multilayer structures have 2 to 7 co-extruded layers comprising at least one antimicrobial coating 22 positioned within the lumen of a tubular support member 24. Multilayer structures comprising two or more antimicrobial materials having different compositions and/or different antimicrobial agents can be formed by coextrusion. Especially preferred end of the stent. Typically, when the diagnostic exam is a PTC, a wire guide and delivery catheter may be inserted via the abdominal wall. If the original exam was an ERCP, the stent may be placed via the mouth. The stent may then be positioned under radiologic, endoscopic, or direct visual control at a point of treatment, such as across the narrowing in the bile duct. The stent may be released using the conventional pushing technique. The delivery catheter may then be removed, leaving the stent to hold the bile duct open. A further cholangiogram may be performed to confirm that the stent is appropriately positioned. Alternatively, other endolumenal medical devices can also be delivered to any suitable body vessel, such as a vein, artery, urethra, ureteral passage or portion of the alimentary canal.

### Hypothetical Examples

An antimicrobial coating may be adhered to a support member formed from polyethylene by dipping the support member in a solution of poly(ethylene-co- vinyl acetate) (EVA) (60% vinyl acetate) in a suitable solvent comprising micronized particles of a Be-containing antimicrobial material comprising 0.2 to 2.8% by weight of beryllium and copper, nickel and/or cobalt. The antimicrobial coating may also comprise poly(styrene-co-isobutylene-styrene) (SIBS) in the solvent. Various amounts of the antimicrobial material may be added to the EVA solution. After removing the coated device from the solution, the coating may then be dried by placing the device in a forced air oven (40°C) for 3 hours. The coated device may then be further dried under vacuum for 24 hours.

An antimicrobial coating solution, such as a polyurethane (CHRONOFLEX 85A) / THF solution (2.5% w/v) and an antimicrobial agent, may be sprayed onto the outer and/or interior surface of the covered stent. The solution may be sprayed at a suitable rate within the lumen of the support member. The coated stent may be allowed to air dry or may be dried under vacuum for a suitable period (eg, 24 hours).

## Claims

1. A fluid drainage device comprising a support member having an interior surface defining a drainage lumen extending along a longitudinal axis of the tubular drainage stent with at least a portion of the interior surface lined with an antimicrobial material including beryllium or a beryllium alloy.

2. The fluid drainage device of claim 1, wherein the antimicrobial material is an alloy comprising beryllium and one or more materials selected from the group consisting of: copper, silicon, cobalt and nickel.

3. The fluid drainage device of at least one of claims 1 and 2, wherein the antimicrobial material is impregnated within a biostable porous material configured to retain the beryllium or beryllium alloy within the tubular drainage stent.

4. The fluid drainage device of claim 3, where the biostable porous material includes a polymer selected from the group consisting of a polyethylene, a polyurethane, an ethylene vinyl acetate copolymer, a copolymer of ethylene with acrylic acid or methacrylic acid.

5. The fluid drainage device of at least one of claims 1-4, wherein the antimicrobial material comprises between about 0.2% and 2.8% beryllium by weight of the antimicrobial material.

6. The fluid drainage device of at least one of claims 1-5, wherein the antimicrobial material comprises an alloy selected from the group consisting of: Be-Cu, Be-Co-Cu, Be-Co-Si-Cu and Be-Ni-Cu.

7. The fluid drainage device of at least one of claims 1-6, where the antimicrobial material is a biostable material.

8. The fluid drainage device of claim 7, wherein the antimicrobial material is a coating comprising two or more layers, the coating being adhered to the interior surface of the support member.

9. The fluid drainage device of claim 8, where the coating comprises a porous layer in contact with the antimicrobial material and defining at least a portion of the drainage lumen.

10. The fluid drainage device of claim 9, where the porous layer has a void-volume percentage of between about 0.25 and 0.95.

11. The fluid drainage device of at least one of claims 9-10, wherein the porous layer is configured to retain a beryllium oxide or a copper oxide within the support member.

12. The fluid drainage device of at least one of claims 8-11, wherein coating comprises a second layer, the second layer comprising beryllium positioned between the interior surface of the support member and the porous layer.

13. The fluid drainage device of at least one of claims 1-12, wherein the support member further comprises a releasable antimicrobial agent.

14. The fluid drainage device of claim 13, wherein the releasable antimicrobial agent comprises one or more materials selected from the group consisting of: cephalosporins, clindamycin, chloramphenicol, carbapenems, minocyclines, rifampin, penicillins, monobactams, quinolones, tetracycline, macrolides, sulfa antibiotics, trimethoprim, fusidic acid, aminoglycosides, amphotericin B, azoles, flucytosine, cilofungin, nikkomycin Z and rifamycin.

15. The fluid drainage device of at least one of claims 1-14, wherein fluid drainage device is a biliary drainage stent including a tubular support member formed from a biostable polymer and the drainage lumen is lined with a biostable antimicrobial material.

16. The fluid drainage device of claim 15, wherein the antimicrobial material includes a mixture of a biostable polymer and the beryllium or beryllium alloy.

17. The fluid drainage device of claim 15, wherein the antimicrobial material comprises an alloy selected from the group consisting of: Be-Cu, Be-Co-Cu, Be-Co-Si-Cu and Be-Ni-Cu.

18. The fluid drainage device of claim 15, wherein the tubular polymer support member is formed from polyethylene, polyurethane or a combination thereof, and the biostable antimicrobial material includes between about 0.2% and 2.8% beryllium by weight of the antimicrobial material and consists of the beryllium, the beryllium alloy or a combination thereof.

19. The fluid drainage device of claim 18, where the biostable antimicrobial material consists of:
a. a porous biostable polymer including at least one of poly(ethylene-co- vinyl acetate) (EVA), poly(styrene-co-isobutylene-styrene) (SIBS), polyethylene and polyurethane; and
b. a beryllium alloy including one or more materials selected from the group consisting of copper, nickel and cobalt.

20. The fluid drainage device of at least one of claims 1-19, where the fluid drainage device is a biliary drainage stent adapted for insertion into a pancreatic duct or a bile duct.

## Patentansprüche

1. Fluiddrainageeinheit, umfassend ein Trägerelement mit einer inneren Oberfläche, die ein Drainagelumen definiert, das sich entlang der Längsachse des schlauchförmigen Drainage-Stents erstreckt, wobei wenigstens ein Teil der inneren Oberfläche mit einem antimikrobiellen Material, das Beryllium oder eine Berylliumlegierung umfasst, belegt ist.

2. Fluiddrainageeinheit gemäß Anspruch 1, wobei das antimikrobielle Material eine Legierung ist, die Beryllium und ein oder mehr Materialien umfasst, die ausgewählt sind aus der Gruppe, bestehend aus Kupfer, Silicium, Kobalt und Nickel.

3. Fluiddrainageeinheit gemäß wenigstens einem der Ansprüche 1 und 2, wobei das antimikrobielle Material in ein biologisch stabiles, poröses Material getränkt ist, das so aufgebaut ist, dass es das Beryllium oder die Berylliumlegierung in dem schlauchförmigen Drainage-Stent hält.

4. Fluiddrainageeinheit gemäß Anspruch 3, wobei das biologisch stabile, poröse Material ein Polymer umfasst, ausgewählt aus der Gruppe, bestehend aus einem Polyethylen, einem Polyurethan, einem EthylenVinylacetat-Copolymer, einem Copolymer von Ethylen mit Acrylsäure oder Methacrylsäure.

5. Fluiddrainageeinheit gemäß wenigstens einem der Ansprüche 1-4, wobei das antimikrobielle Material zwischen etwa 0,2 Gew.-% und 2,8 Gew.-% Beryllium bezogen auf das antimikrobielle Material umfasst.

6. Fluiddrainageeinheit gemäß wenigstens einem der Ansprüche 1-5, wobei das antimikrobielle Material eine Legierung umfasst, ausgewählt aus der Gruppe, bestehend aus Be-Cu, Be-Co-Cu, Be-Co-Si-Cu und Be-Ni-Cu.

7. Fluiddrainageeinheit gemäß wenigstens einem der Ansprüche 1-6, wobei das antimikrobielle Material ein biologisch stabiles Material ist.

8. Fluiddrainageeinheit gemäß Anspruch 7, wobei das antimikrobielle Material eine Beschichtung ist, die zwei oder mehr Schichten umfasst, wobei die Beschichtung an die innere Oberfläche des Trägerelements gehaftet ist.

9. Fluiddrainageeinheit gemäß Anspruch 8, wobei die Beschichtung eine poröse Schicht umfasst, die in Kontakt mit dem antimikrobiellen Material steht und wenigstens einen Teil des Drainagelumens definiert.

10. Fluiddrainageeinheit gemäß Anspruch 9, wobei die poröse Schicht ein prozentuelles Hohlraum-VolumenVerhältnis zwischen etwa 0,25 und 0,95 aufweist.

11. Fluiddrainageeinheit gemäß wenigstens einem der Ansprüche 9-10, wobei die poröse Schicht so aufgebaut ist, dass sie ein Berylliumoxid oder ein Kupferoxid innerhalb des Trägerelements hält.

12. Fluiddrainageeinheit gemäß wenigstens einem der Ansprüche 8-11, wobei die Beschichtung eine zweite Schicht umfasst, wobei die zweite Schicht Beryllium umfasst, das zwischen der inneren Oberfläche des Trägerelements und der porösen Schicht angeordnet ist.

13. Fluiddrainageeinheit gemäß wenigstens einem der Ansprüche 1-12, wobei das Trägerelement ferner ein freigebbares antimikrobielles Mittel umfasst.

14. Fluiddrainageeinheit gemäß Anspruch 13, wobei das freigebbare antimikrobielle Mittel ein oder mehr Materialien umfasst, ausgewählt aus der Gruppe, bestehend aus Cephalosporinen, Clindamycin, Chloramphenicol, Carbaphemen, Minocyclinen, Rifampin, Penicillinen, Monobactamen, Chinolonen, Tetracyclin, Macroliden, Sulfaantibiotika, Trimethoprim, Fusidinsäure, Aminoglycosiden, Amphotericin B, Azolen, Flucytosin, Cliofungin, Nikkomycin Z und Rifamycin.

15. Fluiddrainageeinheit gemäß wenigstens einem der Ansprüche 1-14, wobei die Fluiddrainageeinheit ein Gallendrainage-Stent ist, umfassend ein schlauchförmiges Trägerelement aus einem biologisch stabilen Polymer, wobei das Drainagelumen mit einem biologisch stabilen, antimikrobiellen Material belegt ist.

16. Fluiddrainageeinheit gemäß Anspruch 15, wobei das antimikrobielle Material ein Gemisch aus einem biologisch stabilen Polymer und dem Beryllium oder der Berylliumlegierung umfasst.

17. Fluiddrainageeinheit gemäß Anspruch 15, wobei das antimikrobielle Material eine Legierung umfasst, ausgewählt aus der Gruppe, bestehend aus Be-Cu, Be-Co-Cu, Be-Co-Si-Cu und Be-Ni-Cu.

18. Fluiddrainageeinheit gemäß Anspruch 15, wobei das schlauchförmige Polymerträgerelement aus Polyethylen, Polyurethan oder einer Kombination davon gebildet ist, und das biologisch stabile, antimikrobielle Material zwischen etwa 0,2 Gew.-% und 2,8 Gew.-% Beryllium bezogen auf das antimikrobielle Material umfasst und aus dem Beryllium, der Berylliumlegierung oder einer Kombination davon besteht.

19. Fluiddrainageeinheit gemäß Anspruch 18, wobei das biologisch stabile, antimikrobielle Material aus Folgendem besteht:
(a) einem porösen, biologisch stabilen Polymer, umfassend wenigstens eines von Poly(ethylen-co-vinylacetat) (EVA), Poly(styrol-co-isobutylenstyrol) (SIBS), Polyethylen und Polyurethan; und
(b) einer Berylliumlegierung, umfassend ein oder mehr Materialien, ausgewählt aus der Gruppe, bestehend aus Kupfer, Nickel und Kobalt.

20. Fluiddrainageeinheit gemäß wenigstens einem der Ansprüche 1-19, wobei die Fluiddrainageeinheit ein Gallendrainage-Stent ist, der für die Einführung in einen Pankreasgang oder einen Gallengang ausgelegt ist.

## Revendications

1. Dispositif de drainage de fluide comprenant un élément de support possédant une surface intérieure qui définit une lumière de drainage qui s'étend le long d'un axe longitudinal du stent de drainage tubulaire avec au moins une partie de la surface intérieure recouverte d'un matériau antimicrobien qui comprend du béryllium ou un alliage de béryllium.

2. Dispositif de drainage de fluide selon la revendication 1, dans lequel le matériau antimicrobien est un alliage qui comprend du béryllium et un ou plusieurs matériaux choisis parmi le groupe constitué : du cuivre, du silicium, du cobalt et du nickel.

3. Dispositif de drainage de fluide selon au moins une des revendications 1 et 2, dans lequel le matériau antimicrobien est imprégné d'un matériau poreux biostable configuré pour retenir le béryllium ou l'alliage de béryllium à l'intérieur du stent de drainage tubulaire.

4. Dispositif de drainage de fluide selon la revendication 3, dans lequel le matériau poreux biostable comprend un polymère sélectionné parmi le groupe constitué d'un polyéthylène, d'un polyuréthane, d'un copolymère d'éthylène acétate de vinyle, d'un copolymère d'éthylène avec de l'acide acrylique ou de l'acide méthacrylique.

5. Dispositif de drainage de fluide selon au moins une des revendications 1 à 4, dans lequel le matériau antimicrobien comprend entre environ 0,2 % et 2,8 % en poids de béryllium du matériau antimicrobien.

6. Dispositif de drainage de fluide selon au moins une des revendications 1 à 5, dans lequel le matériau antimicrobien comprend un alliage sélectionné parmi le groupe constitué : du Be-Cu, du Be-Co-Cu, du Be-Co-Si-Cu et du Be-Ni-Cu.

7. Dispositif de drainage de fluide selon au moins une des revendications 1 à 6, dans lequel le matériau antimicrobien est un matériau biostable.

8. Dispositif de drainage de fluide selon la revendication 7, dans lequel le matériau antimicrobien est un revêtement qui comprend deux couches ou plus, le revêtement adhérant à la surface intérieure de l'élément de support.

9. Dispositif de drainage de fluide selon la revendication 8, dans lequel le revêtement comprend une couche poreuse en contact avec le matériau antimicrobien et lequel définit au moins une partie de la lumière de drainage.

10. Dispositif de drainage de fluide selon la revendication 9, dans lequel la couche poreuse possède un pourcentage de volume gazeux situé entre environ 0,25 et 0,95.

11. Dispositif de drainage de fluide selon au moins une des revendications 9 et 10, dans lequel la couche poreuse est configurée pour retenir un oxyde de béryllium ou un oxyde de cuivre dans l'élément de support.

12. Dispositif de drainage de fluide selon au moins une des revendications 8 à 11, dans lequel le revêtement comprend une seconde couche, la seconde couche comprenant du béryllium positionné entre la surface intérieure de l'élément de support et la couche poreuse.

13. Dispositif de drainage de fluide selon au moins une des revendications 1 à 12, dans lequel l'élément de support comprend en outre un agent antimicrobien qui peut être libéré.

14. Dispositif de drainage de fluide selon la revendication 13, dans lequel l'agent antimicrobien pouvant être libéré comprend un ou plusieurs matériaux choisis parmi le groupe constitué : des céphalosporines, de la clindamycine, du chloramphénicol, des carbapenems, des minocyclines, de la rifampicine, des pénicillines, des monobactamines, des quinolones, de la tétracycline, des macrolides, des antibiotiques à base de sulfamides, de la triméthoprime, de l'acide fusidique, des aminoglycosides, de l'amphotéricine B, des azoles, de la flucytosine, de la cilofungine, de la nikkomycine Z et de la rifamycine.

15. Dispositif de drainage de fluide selon au moins une des revendications 1 à 14, dans lequel le dispositif de drainage de fluide est un stent de drainage biliaire qui comprend un élément de support tubulaire formé à partir d'un polymère biostable et la lumière de drainage est recouverte d'un matériau antimicrobien biostable.

16. Dispositif de drainage de fluide selon la revendication 15, dans lequel le matériau antimicrobien comprend un mélange d'un polymère biostable et le béryllium ou l'alliage de béryllium.

17. Dispositif de drainage de fluide selon la revendication 15, dans lequel le matériau antimicrobien comprend un alliage sélectionné parmi le groupe constitué : du Be-Cu, du Be-Co-Cu, du Be-Co-Si-Cu et du Be-Ni-Cu.

18. Dispositif de drainage de fluide selon la revendication 15, dans lequel l'élément de support tubulaire en polymère est formé à partir de polyéthylène, de polyuréthane ou d'une combinaison de ceux-ci, et le matériau antimicrobien biostable comprend entre environ 0,2 % et 2,8 % en poids de béryllium du matériau antimicrobien et est constitué du béryllium, de l'alliage de béryllium ou d'une combinaison de ceux-ci.

19. Dispositif de drainage de fluide selon la revendication 18, dans lequel le matériau antimicrobien biostable est constitué de :
a. un polymère biostable poreux comprenant au moins un élément parmi le poly(acétate d'éthylène-co-vinyle) (EVA), le poly(styrène-co-isobutylène-styrène) (SIBS), le polyéthylène et le polyuréthane ; et
b. un alliage de béryllium comprenant un ou plusieurs matériaux sélectionnés parmi le groupe constitué du cuivre, du nickel et du cobalt.

20. Dispositif de drainage de fluide selon au moins une des revendications 1 à 19, dans lequel le dispositif de drainage de fluide est un stent de drainage biliaire adapté pour insertion dans un canal pancréatique ou biliaire.
